(19)

Europäisches
Patentamt
European
Patent Office
Office européen
des brevets

(11) **EP 2 298 788 A1**

(12) **EUROPEAN PATENT APPLICATION**

(43) Date of publication:
**23.03.2011 Bulletin 2011/12**

(21) Application number: **10180934.1**

(22) Date of filing: **01.10.2002**

(51) Int Cl.:
*C07K 1/113* [(2006.01)]  *C07K 1/16* [(2006.01)]
*C07K 1/18* [(2006.01)]  *C07K 1/20* [(2006.01)]
*C07K 1/22* [(2006.01)]  *C07K 1/36* [(2006.01)]

(84) Designated Contracting States:
**AT BE BG CH CY CZ DE DK EE ES FI FR GB GR
IE IT LI LU MC NL PT SE SK TR**

(30) Priority: **03.10.2001 EP 01123698**

(62) Document number(s) of the earlier application(s) in
accordance with Art. 76 EPC:
**02800587.4 / 1 438 327**

(71) Applicant: **Boehringer Ingelheim RCV GmbH & Co
KG
1121 Wien (AT)**

(72) Inventors:
• **Necina, Roman
55216, Ingelheim am Rhein (DE)**
• **Schlegl, Robert
55216, Ingelheim am Rhein (DE)**
• **Jungbauer, Alois
55216, Ingelheim am Rhein (DE)**
• **Machold, Christine
55216, Ingelheim am Rhein (DE)**

(74) Representative: **Hammann, Heinz et al
Boehringer Ingelheim GmbH
Corporate Patents
Binger Strasse 173
55216 Ingelheim am Rhein (DE)**

Remarks:
This application was filed on 28-09-2010 as a
divisional application to the application mentioned
under INID code 62.

(54) **Method for reconstituting a recombinant protein to its biologically active form**

(57) In a method for reconstituting a recombinant protein from a denatured state to its active form, a feed solution containing the recombinant protein in its denatured and/or in biologically inactive intermediate forms is subjected to a chromatographic separation process, in which the protein is reconstituted under conditions that promote refolding of the protein and the intermediate forms are separated from the refolded protein. The denatured form and/or the inactive intermediate forms of the protein are separated from the refolded protein in a continuous or quasi-continuous manner and optionally recycled to the feed solution.

**Fig.3**

**Description**

The invention relates to the field of recombinant protein production.

Proteins for industrial applications, e.g. for use as biopharmaceuticals or fine chemicals, are either obtained by extraction and purification from a natural source, such as a plant or animal tissue or microorganisms, or by means of recombinant DNA technology.

To produce a recombinant protein, the cDNA encoding the protein of interest is inserted into an expression vector and the recombinant vector is transformed into host cells, which are grown to overexpress the protein. The host cells may be selected from microorganisms such as bacteria, yeast or fungi, or from animal or plant cells.

Overexpression of a protein is a complex event. To obtain the correct conformation, the protein, already in its native state, is associated with so-called "folding helper proteins" and enzymes. The folding helper proteins, also termed "chaperones" or "minichaperones", interact in a complex way so that the protein regains its native conformation after passing through various intermediate states. Some of the intermediate states may be quite stable. Enzymes involved in protein maturation either catalyze the rapid formation of disulfide bridges (1; 2), the isomerization of prolyl-peptide linkages (3-6) or more complex modifications, such as the truncation of the protein, side chain modifications or modifications of the N-and C-terminus. When a protein is efficiently overexpressed, the production of the nascent peptide chain occurs faster than the folding of the protein. For some proteins, an intermediate state may also form aggregates (in the following, the term "intermediate" forms also encompasses aggregate forms).

Overall, aggregate formation occurs much faster than the complete folding of a protein (7; 8).

In expression systems, in which such conditions are present, the protein is deposited in the cells in a paracrystalline form, so-called "inclusion bodies", also termed "refractile bodies".

Since the protein, when present in the form of insoluble inclusion bodies, is shielded from enzymatic attack, such as proteolysis, and cannot interfere with the physiology of the cells. Recombinant DNA technology has taken advantage of this aberrant way of protein secretion, e.g. for the production of the proteins that are toxic for the cells.

To obtain a protein from host cells, in which it is accumulated in a denatured form, i.e. a conformational state without biological activity, various steps have to be taken to obtain the protein in its correctly refolded form. For example, bacterial cells carrying inclusion bodies are disintegrated, the inclusion bodies harvested by centrifugation and then dissolved in a buffer containing a chaotropic agent. The denatured protein is then transferred into an environment that favours the recovery of its native conformation. Before adopting its native state, the protein undergoes a transition through various semi-stable intermediates. Since intermediates have highly exposed hydrophobic domains, which are prone to associate, they tend to form aggregates. In principle, refolding may be considered as a race against aggregate formation, which usually follows second order reaction kinetics, while refolding of the protein follows first order reaction kinetics (8).

With the currently available methods, refolding of proteins is achieved by diluting the protein in a refolding buffer in a batch or continuous mode (9-13). In these methods, batchwise dilution results in highly diluted protein solutions and therefore large process volumina, which often is the bottleneck in industrial processes.

In another approach the folding pathway is simulated *in vivo* by adding chaperons and/or minichaperons, and/or enzymes that catalyze certain steps in the folding pathway (2; 14-18). Complex refolding reactor systems comprising series of tanks have been designed to improve the refolding reaction (19).

In another approach, the helper proteins and enzymes are immobilized to a solid phase. Then the protein solution is passed over a so-called Packed Bed containing the immobilized helper proteins and/or helper enzymes, thus being folded into its native conformation (20-23). Since the folding helper proteins and enzymes must be present in a stoichiometric ratio, this process requires almost the same amount of helper proteins, which in turn have to be produced by recombinant DNA technology, as the finally obtained product. In addition, to improved folding, the helper proteins are usally fused to the protein of interest, which requires further processing of the fusion protein. For these reasons, this strategy is very cost intensive.

Since a certain protein fraction is lost in the form of aggregates, refolding of the protein in free solution or in the matrix-assisted process is not efficient enough to transfer the denatured protein into the folded form in a quantitative way.

A protein can be refolded from its denatured conformation to the correctly folded conformation by transferring it into an environment that favors the change to the native conformation. During this rearrangement, the protein passes through several intermediate conformational states, which are prone to form aggregates. Depending on the individual protein and on the environmental conditions, the aggregates may precipitate. Independent of whether the aggregates remain soluble or whether they precipitate, this process leads to dramatic losses in the yield of correctly folded protein. In general, the folding of a protein to its native conformation follows first order reaction kinetics, while the formation of aggregates from intermediates follows second or higher order reaction kinetics.

It was an object of the invention to provide an efficient method for refolding a protein from a denatured state, which overcomes the shortcomings of the currently used methods and which can be operated without using helper proteins.

The solution of the problem underlying the invention is based on the consideration that the chromatographic separation process may be improved by running it continuously. In addition, it was hypothesized that recycling the intermediate forms of the protein may further allow both to improve the yield of a recombinant protein and to work at high protein concentrations, which would significantly reduce the process volume.

The present invention relates to a method for obtaining a biologically active recombinant protein by reconstituting the protein from a denatured state to its active form, wherein a feed solution containing the recombinant protein of interest in its denatured and/or in biologically inactive intermediate forms is subjected to a chromatographic separation process, in which the protein is reconstituted under conditions that promote refolding of the protein and the intermediate forms are separated from the refolded protein, **characterized in that** the denatured and/or in inactive intermediate forms are separated from the refolded final product in a continuous or quasi-continuouschromatographic method.

The term "denatured form", in the meaning of the present invention, designates the biologically inactive form of the expressed protein of interest, as obtained as a product of the recombinant production process, usually as obtained after dissolving the inclusion bodies.

The term "intermediate forms" or "intermediates" in the meaning of the present invention, designates the forms that the protein passes through between its denatured form and its reconstituted (refolded) native and biologically active state. The intermediates, which are biologically inactive or have a lower biological activity than the native protein, may be in the form of aggregates. (The term "inactive", in the context with intermediate forms, also encompasses forms of the protein with a lower activity as compared to the biologically fully active form of the protein.)

In a preferred embodiment, the intermediate forms that have been separated form the refolded protein are reintroduced (recycled) into the feed solution and thus undergo the reconstitution process at least one additional time. The intermediates, when separated by the process of the invention, may still contain a fraction of denatured protein.

In order to keep the chromatography medium, usually a gel, properly working, it has to be regenerated. This is achieved by a regeneration solution that is applied to the packed bed. Depending on the chemical properties of the chromatography medium, this solution may be either a strong alkaline solution or a strong acidic solution, or a chaotropic buffer, or an organic solvent, e.g. ethanol, or an aqueous buffer supplemented with an organic solvent, or an aqueous buffer with an ionic or non-ionic detergent. The regenerating solution (regenerate) must be able to remove irreversibly bound protein fractions from the chromatography medium. The regenerate may be the feed solution itself or it may different from the feed solution and applied to the chromatographic medium separately from the feed solution.

In the case that the regenerate that exits the chromatographic process contains a significant amount ($\geq 10\%$) of intermediates, it is, in a preferred embodiment, recyled to the process, either separately or by combining it with the eluate stream that contains the intermediates.

In a particularly preferred embodiment, the eluate and/or regenerate containing the intermediate forms can be concentrated and/or diafiltrated before it is reintroduced into the starting feed solution. Thereby, yield and productivity of the refolding process is further improved. Concentration may be achieved by conventional means, e.g. by ultrafiltration (in the case of soluble intermediates) or microfiltration (in the case of insoluble intermediates/ aggregates).

In the following, referring to the protein, the term "refolding" is used for "reconstituting from a denatured state to its active form".

The (starting) feed solution is the solution that has been obtained from fermentation of bacterial, yeast, fungal, plant or animal cells carrying an expression vector encoding a heterologous protein of interest.

In the present invention, the feed solution is usually obtained from conventional microbiological fermentation. The feed solution contains the recombinant protein in the solubilized form as obtained from the inclusion bodies.

The feed solution contains, besides buffer substances, components that promote the dissociation of the recycled aggregates, e.g. chaotropic agents such as urea, guanidinium chloride (GuHCl), sodium and/or potassium thiocyanate, and reducing agents such as mercaptoethanol, dithiothreitol, monothiogylcerol. Typical compositions and conditions are known in the art, they have been extensively described in the literature (11; 12; 58). In the case of size exclusion chromatography, a feed solution containing denaturing and/or reducing agents, may, at the same time, serve as a regenerating solution.

Starting from a given feed solution, the person skilled in the art is familiar with the measures that have to be taken to provide the conditions that promote refolding, i.e. appropriate refolding environment, during the chromatographic process of the invention. First, to obtain conditions that promote refolding of the protein, the chaotropic and/or reducing agents required for solubilization of the inclusion bodies and denaturation of the protein that are contained in the feed solution have to be removed, either completely or to an extent that is tolerated by the protein. In the present invention, this is achieved during the chromatographic process by washing out the above-mentioned agents with a suitable refolding buffer, e.g. a Tris or phosphate buffer, such that an optimal refolding environment in terms of pH, conductivity and temperature is given (11, 12, 58).

The feed solution may be diluted to achieve partial refolding of the protein before it undergoes the chromatographic refolding process.

To ensure the optimal conditions for refolding, the refolding buffer may be supplemented by agents that provide the optimal redox potential and thus promote the correct formation of disulfide bridges, e.g. oxidized and reduced glutathione or cystine/cysteine, and/or agents that prevent aggregation, e.g. L-arginine, urea, polyethyleneglycol (11, 12, 58).

The separation of the intermediates from the correctly folded protein can be accomplished by any continuous or quasi-continuous chromatographic method that has been proven useful for the separation of proteins.

A great number of standard chromatographic methods that are routinely used for protein separation are known from the literature, most of them being applicable on commercially available devices, e.g. chromatographic columns. Depending on the principle of separation, the methods are divided into ion-exchange chromatography, affinity chromatography, hydrophobic interaction chromatography, reversed phase chromatography, covalent chromatography, size exclusion chromatography or adsorption chromatography.

In principle, these chromatographic methods can be conducted in a batchwise, a quasi-continuous or a continuous mode of operation. In the batchwise mode of operation, the feed solution is loaded onto a chromatographic support, e.g. a packed bed or an expanded bed, and the protein, depending on its affinity to the stationary phase, is either strongly retained or passes through the column. In the case the protein is strongly retained, it can be desorbed by a change of running conditions after the unbound material has been washed out.

The batch process can be transferred to a quasi-continuous mode either by working with several columns in a sequential manner or by placing the columns in a manifold of valves to allow a continuous operation, termed "carousel chromatography".

Any chromatographic protein separation method, provided it can be conducted in a quasi-continuous or continuous mode, can be used in the present invention. The person skilled in the art is familiar with these methods and can select the most appropriate one to a given separation requirement.

Annular chromatography (AC), carrousel chromatography, simulated moving bed (SMB) and true moving bed (TMB) chromatography are the most widely used chromatographic separation systems that are operated in a continuous or quasi-continuous manner. The advantages of AC over SMB and TMB lie in the application of a gradient elution and the separation of multi-component mixtures (24).

In a preferred embodiment, the method of the invention is annular chromatography. This method has been suggested for a great variety of separation problems ranging from small molecules to biopolymer separations (25-51). The original concept of an annular chromatograph, as proposed by Martin (59) and realized by Fox et al. (52-54), was further developed at the Oak Ridge National Laboratory to operate the system under a certain pressure (25; 38; 49). The Pressurized-Continuous Annular Chromatograph (P-CAC) was designed as a closed system. Two concentric cylinders form an annulus, into which the chromatography medium is packed. Feed and eluent (which is, in the method of the invention, the refolding buffer) are introduced in a continuous way at the top of the bed. The entire bed slowly rotates, while the feed solution is introduced from a stationary entry and the eluent is uniformly present everywhere else around the annulus. The separation of the feed solution into single components is caused by the rotation of the sorbent. The separated components appear as helical bands, each of which has a characteristic, stationary exit point. Three factors have an effect on the location of the exit point: (a) eluent velocity, (b) rotation rate of the annulus, and (c) the distribution coefficient.

Any annular chromatographic method and device can be used in the present invention. Examples of annular chromatographs that are suitable for use in the present invention are described in the literature (see the previous paragraph) and in the following patent applications: WO 98/45699, WO 99/28740, WO 01/388866, EP 1 134 581. The devices can be obtained commercially, e.g. from Prior, Götzis, Austria.

In the case of using annular chromatography in the method of the invention, the annulus is packed with a special chromatography medium allowing separation according to either ion-exchange chromatography, affinity chromatography, hydrophobic interaction chromatography, reversed phase chromatography, covalent chromatography, size exclusion chromatography or adsorption chromatography. The choice of chromatographic principle depends on structure of the protein, the concentration of the protein, the amount and nature of the contaminants, the overall process flow scheme and the availability of a particular protein ligand (in the latter case, the method of choice usually is affinity chromatography).

The feed solution containing the denatured protein (and optionally spontaneously formed intermediates and aggregates) is continuously fed to the rotating annulus that is packed with the chromatography medium. The medium, usually a gel, is perfused with a buffer promoting refolding of the protein (the refolding buffer, as described above). While the proteins pass through the column, the refolding process takes place. Due to their physicochemical properties (such as molecular size, hydrophobicity, accessible charged and hydrophobic groups, solubility etc.) of the intermediates, the native refolded protein and the intermediates are retained differently. According to their different retention properties, the different states of the protein, i.e. the intermediates and the biologically active protein, respectively, elute at different exit points of the annulus. In a preferred embodiment, the exit stream containing the intermediates, possibly in the form of soluble or suspended aggregates, is collected and recycled to the feed solution. To increase the amount of intermediates/aggregates, they can be concentrated by any suitable concentration device, e.g.

a tangential flow ultrafiltration unit. The optionally concentrated, recycled intermediates/aggregates become then components of the feed solution and undergo at least one additional refolding process.

In an alternative embodiment that uses annular chromatography, the denatured protein is adsorbed and completely or partially refolds during the adsorption process, which occurs in the presence of refolding buffer. The conditions for subsequent desorption must also favour refolding, i.e. they have to ensure, on the one hand, that the protein that has already refolded during adsorption maintains its native conformation and, on the other hand, that refolding of the remaining protein is promoted. The protein fractions, which have not refolded, can be separated from the refolded protein during the adsoption and/or desorption step and are, in a preferred embodiment, recycled by adding them to the feed solution.

The annular chromatography process results in a stoichiometric conversion of the denatured protein to its correctly folded native state. Another advantage is a decrease in process volume and the possibility to maintain a continuous process. In order to keep the chromatography gel properly working, it has to be regenerated. A regeneration solution is applied to the packed bed at a position distant enough from the feed inlet position to avoid mixing of the regeneration solution with the feed solution. The composition of the regenerating solution depends on the chemical properties of the chromatographygel, as described above. The regenerate eluates at a position different from the eluate containing the intermediates and the native, refolded protein. The regenerate can be, in case that it contains significants concentrations of protein, recyled. This may be done by re-introducing it into the starting feed solution by iteself or by combining it with the eluate containing the intermediates.

In another preferred embodiment, the chromatographic method used in the method of the invention is the Simulated Moving Bed (SMB) process, which was first developed in the early sixties by the Universal Oil Product Company (55; 56). It was mainly applied to industrial scale separations, such as the separation of xylenes or the separation of fructose and glucose. By employing a suitable system of adsorbant and eluate, a feed stream is separated into two withdrawal streams containing the pure components of a binary or pseudo-binary mixture (a mixture of two or more compounds that, due to their different physico-chemical properties, can be divided into two fractions). The SMB process divides a large column into a finite number of small sections, also termed "zones", between which withdrawal tubes are situated. These tubes are connected with the inlets and outlets in a cyclic mode via a specially designed rotary valve. Switching the rotary valve at a defined point of time simulates a countercurrent flow of solid and fluid phase. Hidajat et al. 1986 (57) have shown that the SMB is equivalent to TMB. For SMB applications one large column may be used or the large column may be substituted by a number of smaller columns. There are inlets or outlets for the feed solution, the eluent buffer, extract and raffinate, called nodes, dividing the arrangements of columns into four zones. Special valves allow the liquid to flow in only one direction. The inlets and outlets are arranged in a predefined manner. These nodes are switched in the same direction as the fluid flows or the columns are switched counterwise to this direction at a defined time interval. As a result, there is a countercurrent flow of solid and fluid phase.

In the SMB mode, the method of the invention is operated in the quasi-continuous mode and preferably carried out as follows:

At time to the feed solution containing the denatured protein (and optionally intermediates) is continuously injected between zones II and III. The zones are defined analogously to the true moving bed; at zone I the liquid is introduced, between zone I and II the extract is collected, between zone II and III the feed solution is introduced, between zone III and IV the raffinate is collected. Hereby component A (the aggregates) is defined as the least adsorbable fraction, and component B (the refolded protein including the intermediates and additional contaminating proteins) as the more strongly retained component. The feed solution is pushed into zone III by the eluent (refolding buffer). Component A (least adsorbable) migrates faster than component B (strongly adsorbed or retained). Before component A reaches zone IV, a part of the protein solution is withdrawn by the raffinate outlet. The remaining part is transported into zone IV.

Just before the front of component B reaches the raffinate outlet, the inlets and outlets have to be switched to the next position to avoid a contamination of raffinate. The switching has to be in the same direction as the liquid flow, while the column remains stationary in space. The saturated columns in zone II are cleaned by fresh eluent. The mixture flowing out from zone II is mixed with the feed solution and transported into zone III. In this section, component A is displaced by component B. The faster migrating component A reaches the raffinate outlet again.

Before the breakthrough of the component B at the raffinate outlet point, there is a switching into the 3rd state. A full cycle is completed after the fourth switching, assuming the simplest configuration of a SMB or TMB.

There is an apparent rotation of the columns of 360°. The cyclic steady state of the system is reached after several full cycles. At the extract and raffinate outlet, the desorption and the breakthrough fronts of the components A and B can be collected. The intermediates (component B) are continuously recycled to the feed solution.

In a continuous mode of operation the protein concentration of the eluate stream is constant over time, e.g. in annular chromatography at a given exit. In contrast, in the quasi-continuous mode it changes cyclically over time, e.g.in SMB due to the distinct zones.

In its extreme form, a continuous separation process is divided into an indefinite number of separation sections.

As to the refolding environment during the chromatographic process, for many industrially useful proteins guidance for defining the parameters that promote refolding are available in the literature (11; 12; 58). For a novel protein of interest, depending on the specific protein, given a feed solution from a fermentation process, the refolding parameters can be determined and optimized in serial experiments by performing dilution experiments in a small-scale batch mode. These experiments can be conducted by varying the refolding buffer with regard to the above listed factors, e.g. pH, redox potential, etc. The obtained conditions are transferred on a chromatography column. The elution positions for the various protein forms, i.e. the refolded, intermediate and aggregate forms, are determined. The suitable chromatographic process is then designed on the basis of these values.

Similarly to the composition of the refolding buffer, the other chromatographic process parameters, e.g. feed flow rate, eluent flow rate, feed concentration, column length and diameter, temperature etc. can be determined and optimized depending on the individual protein. A prerequisite for the separation is that it has a different selectivity for the aggregates, the intermediates and the refolded form of the protein. The aggregated forms and the intermediates differ from the native molecule at least in size, hydrophobicity and charge.

For the preferred embodiment of the invention, in which the eluent containing intermediates is recycled, the re-circulation (recycling) ratio is adjusted depending on the mode of chromatographic separation. For adsorptive separation methods such as ion exchange chromatography and adsorption chromatography, the feed solution can be diluted to any extent with the solution containing the recycled aggregates. The re-circulation (recycling) ratio depends on the eluate stream containing the intermediates/aggregates. In size exclusion methods, the volume of recycled feed is critical, because in these methods the separation is strongly effected by the feed volume and flow rate of the eluent. In these methods, care needs to be taken that the amount of feed solution should never exceed one third of the total column volume. For critical separation problems, this amount is even lower. Thus a concentration step has to be inserted after collection of the eluted intermediates/aggregates. This can be achieved by a conventional ultrafiltration system.

In another preferred embodiment, the method of the invention is carrousel chromatography. Suitable devices are commercially available, e.g. from SepTor Technologies BV, Utrecht. The Septor is a carrousel type quasi-continuous system. In order to transport the chromatography columns through all steps in the process cycle, they are mounted on a slowly rotating carousel. The carrousel typically rotates clockwise and includes all process steps as applied in a conventional chromatography step (equilibration, loading of the feed solution, washing, elution, regeneration). In order for the columns to move along all different sections in the process cycle, the columns are connected to a multiport indexing valve. The stream of the eluate containing the intermediates/ aggregates is preferably recycled to the feed stream. Prior to recycling, the eluate solution is preferably concentrated.

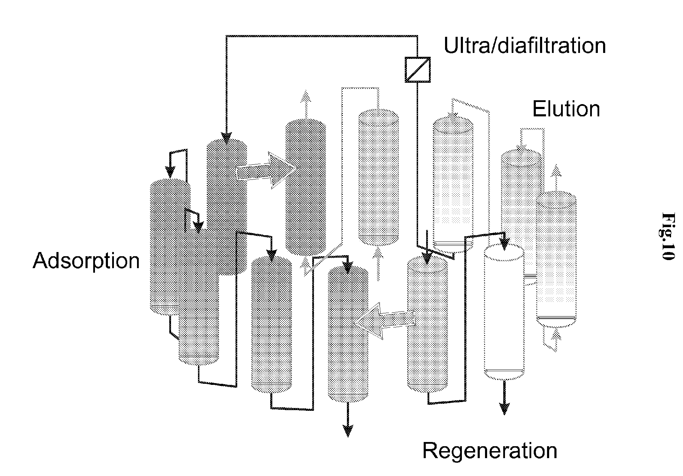

Figure 10 shows a typical scheme of caroussel chromatography.

Overall, the method of the invention has the following advantages: it allows for high protein concentrations in the feed solution, it is highly efficient in terms of refolding yield, it allows for separation of the refolded protein from the intermediates and it can be operated without employing chaperons. (However, the method of the invention does not exclude the use of chaperons, which may be immobilized on the chromatographic column, which thus operates as a catalytic refolding reactor, or which may applied as components of the refolding buffer). The method of the invention can be operated from small laboratory scale to industrial scale. The chromatographic devices used in the method of the invention are commercially available and can be, in terms of size and performance, supplied from the manufacturers according to the customer's needs; for recombinant proteins, the required capacities usually range from a few mg to kg amounts.

Brief description of the figures

Figure 1: Refolding of denatured and reduced $\alpha$-lactalbumin by size exclusion chromatography on Superdex 75 PrepGrade column at a linear velocity of 30 cm/h.

Figure 2: Continuous refolding of denatured $\alpha$-lactalbumin by annular size exclusion chromatography on Superdex 75 PrepGrade column at 250°/h and 30 cm/h.

Figure 3: Schematic drawing of experimental setup for continuous refolding by annular chromatography and recycling of aggregates

Figure 4A: Reversed phase HPLC chromatogram of fractions from matrix assisted refolding with HIC

Figure 4B: Reversed phase HPLC chromatogram of native $\alpha$-lactalbumin

Figure 4C: Reversed phase HPLC chromatogram of denatured and reduced α- lactalbumin

Figure 5: Matrix assisted refolding of rHuAFP on Sephacryl 200HR

Figure 6: Capture step of refolded rHuAFP on Q- Sepharose XL

Figure 7: SDS-PAGE (silver stain) of native protein fraction eluted from Q- Sepharose XL

Figure 8: Analytical size exclusion chromatography of native protein fraction eluted from Q- Sepharose XL

Figure 9: Continuous refolding of denatured and reduced α-lactalbumin by annular ion exchange chromatography

Figure 10: Schematic illustration of caroussel chromatography

Example 1

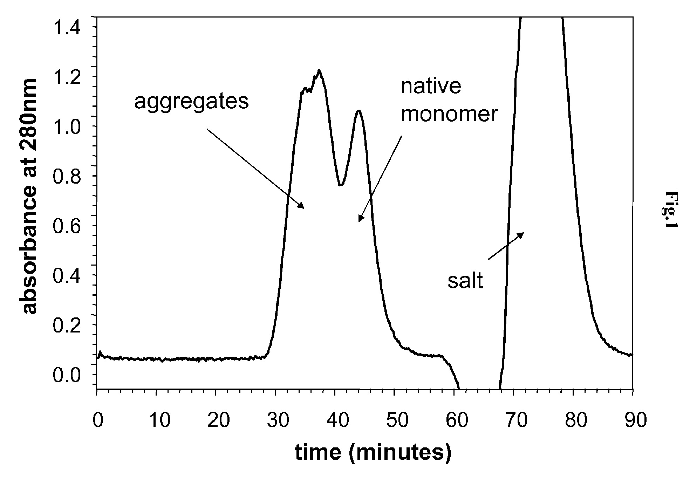

Continuous refolding of α-lactalbumin by matrix-assisted refolding on gel-permeation chromatography

a) Before transferring the process into a continuous mode, refolding was tested on a conventional packed bed using a Superdex 75 PrepGrade column from AP biotech (Uppsala, Sweden). α-lactalbumin was dissolved in a 50mM tris buffer, pH 8.5 supplemented with 6 M GuHCl and 20mM dithiothreitol. These conditions induce complete denaturation of protein and splitting of the disulphide bridges into free sulfhydryl groups. The protein concentration was 3.7 mg/ml. A Superdex 75 PrepGrade column with 1.6 cm i.d. and 37 cm height was packed and 1ml feed (reduced α-lactalbumin) was injected after the column had been equilibrated with a 50mM Tris buffer supplemented with 2mM cysteine, 2mM cys- tine and 10mM $CaCl_2$ at a flow rate of 30 cm/h. While passing through the column, the refolded proteins were separated from the aggregates (Fig. 1). The column effluent was continuously monitored at 280 nm. The native protein and the aggregates were analyzed by analytical size exclusion chromatography and RP-HPLC. The refolding yield was about 26 percent. The addition of 0.25M L-Arginine into the refolding-buffer increased the yield to 40%.

b) For the continuous refolding experiments, the same protein solutions and buffers were used. The Superdex 75 PrepGrade chromatography medium was packed onto a annular chromatography System, PCAC from Prior Separation Technology (Götzis, Austria). The PCAC consists of two concentric cylinders forming an annulus into which the stationary phase is packed. The outer cylinder had a diameter of 15 cm and the inner one a diameter of 14 cm, resulting in an annulus width of 0.5 cm. The upper part of the outer cylinder is made of glass and the lower part of polypropylene. The inner cylinder is made of polypropylene and is shorter than the outer one, leaving a head space at the top. Both cylinders are closed by a head from PEEK (Polyetheretherketone) through which the eluent and feed streams are inserted. The feed stream was pumped at the top of the gel bed through a fixed feed nozzle, whose tip was located within the layer of the glass beads. At the bottom of the unit, the two cylinders are attached to a stainless steel plate which contains 90 exit holes covered by a nylon filter (11 μm pore size). The bot- tom of the rotating column is connected to a fixed teflone slip-ring which also contains 90 exit ports connected to a short section of Tygon tubing (Norton Performance Plastic Corporation, Akron, Ohio, USA). The exit ports are evenly distributed at 4° intervals along the annulus. The column was packed to a height of 4 1 cm cm with Superdex 75 Prep- Grade. The bed of the glass beads was 2.6 cm high.

The system was additionally equipped with a pump for recycling the aggregates to the feed solution. The transfer of a batchwise separation into a continuous one is made by transformation of the elution time (t) and angular velocity (ω) into angular displacement (θ).

$$\theta = \omega^* t$$

From this calculation, the exit point of the various separated components can be determined.

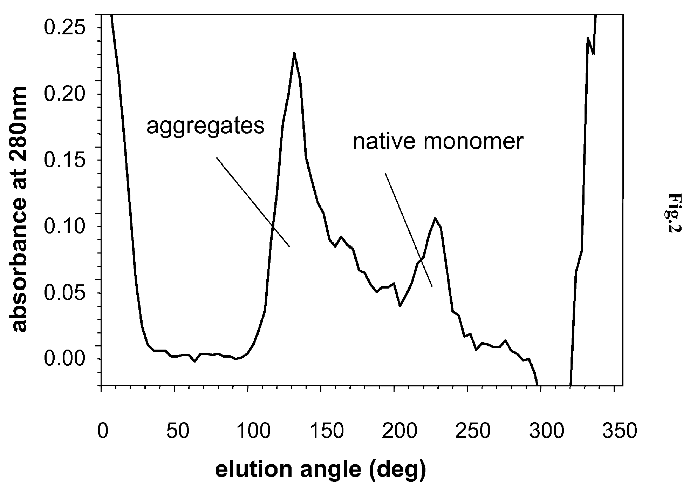

Next, the refolding process was performed continuously. A rotation rate of 250°/h was applied and the eluent flow rate was 30 cm/h. A feed flow rate of 0.31 ml/min was applied. The chromatogram obtained after continuous refolding of lactalbumin by size exclusion chromatography is shown in Figure 2.

After separation had reached a steady state, collection of the fractions containing the aggregates was started. Continuous concentration commenced when 50 ml were collected. Samples were drawn and the amount of aggregated protein and native protein was determined.

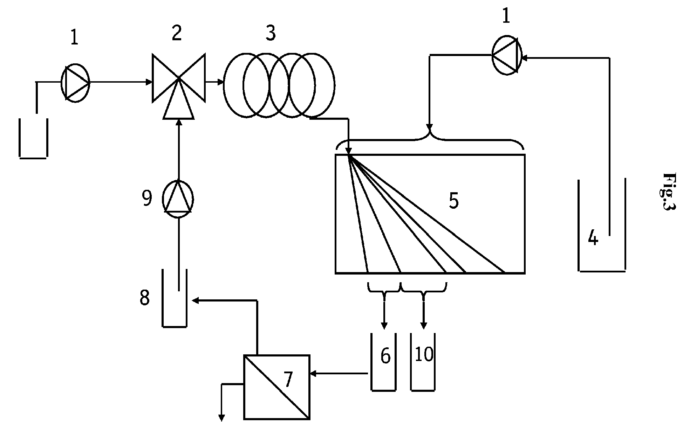

The effluent of those ports where the aggregates are eluted was collected continuously and concentrated by tangential flow filtration using a Millipore tangential flow filtration system with Biomax 5K membranes. The concentration was adjusted to approx. 1 mg/ml. A schematic drawing of the experimental setup for continuous refolding by annular chromatography and recycling of aggregates is shown in Figure 3:

1 is the feed pump delivering the reduced lactalbumin, 2 is the mixer for blending of fresh feed with recycled feed after concentration by tangential flow filtration. 3 is the reaction loop to complete reduction of recycled aggregates; 4 is the eluent pump for the annular chromatography system, 5 is the annular chromatography system, 6 a collecting device consisting of a simple glass bottle, 7 a tangential flow filtration device, 8 a vessel for collection of concentrated aggregates, 9 is the recycling pump and 10 a vessel for collection of refolded protein.

After the system had reached equilibrium, the refolding efficiency at a protein concentration of 3.7 mg/ml was raised from 26% without recycling to > 80% with recycling.

Example 2

Continuous refolding of human alpha-fetoprotein (rHuAFP) with the annular chromatography system PCAC and estradiol sepharose

Human alpha-fetoprotein was expressed in Escherichia coli as described by Boismenu et al. (60) The cells were expanded in 10 x 5 L shake flasks and harvested by a bucket centrifuge The resuspended cells were disintegrated by a high pressure homogenizer at 500 bar. The homogenate was clarified by centrifugation at 10.000g and the sediment containing the inclusion bodies was dissolved in 6M urea by excessive stirring. This solution was partially refolded by dilution. The partially refolded solution was further processed by a continuous adsorption/desorption on estradiol sepharose. The estradiol sepharose was prepared as described by Feng et al. (61) and packed into the annular chromatograph. The same annular chromatograph as in Example 1 was used. The refolded rHuAFP was bound to the estradiol sepharose and could be eluted in a concentrated form, while the non-refolded part was found in the flow through and recycled to the feed solution. In order to avoid excessive dilution of the feed, the recycled solution was concentrated by tangential flow filtration and urea was added to supplement for chaotropic activity in the feed solution.

Example 3

Continuous refolding of a-lactalbumin on hydrophobic interaction chromatography (HIC) sorbents

Bovine a-lactalbumin was dissolved in 50 mM Tris/HCl, 10 mM CaCl$_2$, pH 7.0, and denatured with 6 M GuHCl and 250mM β-mercaptoethanol. This was performed at a concentration of 5 mg/ml at room temperature. The refolding took place at the HIC sorbent. As an example Macroprep Methyl from BioRad (Hercules, CA, USA) was chosen. Prior to the loading of the denatured lactalbumin the column had been equilibrated with 1.5 M ammonium sulfate. The ammonium sulfate was dissolved in a 50mM Tris/HCl, 10mM CaCl$_2$, 2mM cysteine/ cystine buffer pH 7.0. Solid ammonium sulfate was added to the denatured protein solution to reach a final concentration of 1.5. M. Then 2.5 ml of the denatured protein solution supplemented with ammonium sulfate was loaded on a 14cm x 1.0 cm i.d. methyl Sepharose column at a linear velocity of 100 cm/ h. The column effluent was monitored at 280 nm. A residence time of 25 min of protein was sufficient to elute refolded protein with a 50 mM Tris/HCl, 10mM CaCl$_2$, 2mM cysteine/cystine buffer pH 7.0. The column was regenerated with 20% ethanol dissolved in water. This peak contained residual α-lactalbumin in the unfolded state.

Refolding was examined by reversed phase HPLC (Vydac C4, 214TP54). Fully denatured α-LA was separated from oxidative folding intermediates and native protein by linear gradient elution from 37% to 45% acetonitril/water containing 0.1% TFA in 15 minutes at 1 ml/min and 30°C.

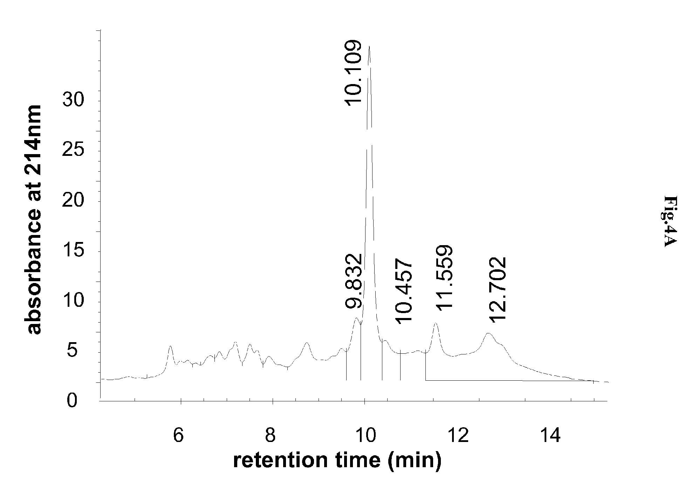

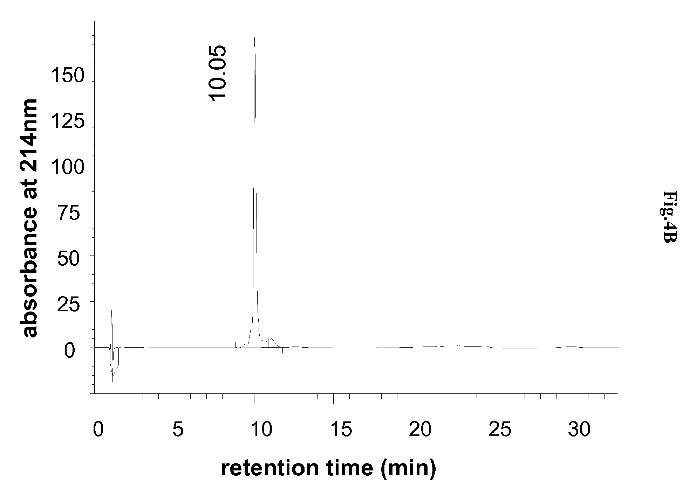

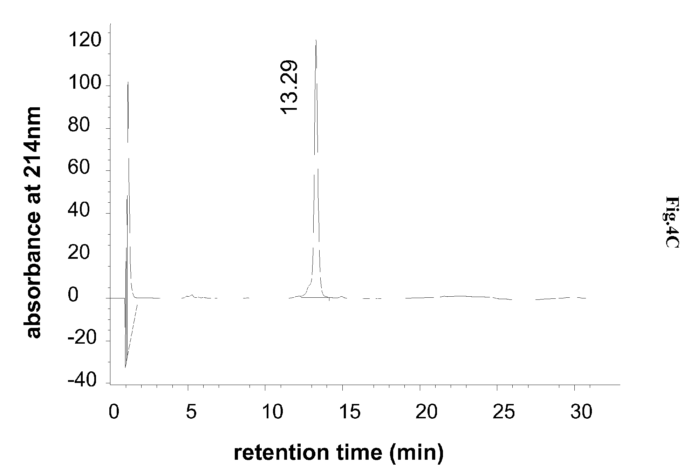

All runs were performed on an Agilent LC 1100 system. A Reversed phase HPLC chromatogram of fractions containing refolded protein after matrix assisted refolding with HIC is shown in Fig. 4A. RP-HPLC chromatograms of native and denatured/reduced α-lactalbumin are shown in Fig. 4B and 4C, respectively.

These refolding conditions were transferred to continuous annular chromatography. Macroprep Methyl medium was packed into a annular chromatograph from Prior Separations Technologies (Götzis Austria). The system is described in Example 1. A column height of 14 cm was chosen and the annulus width was 0.5 cm. At positions 0-12 ° the denatured α-lactalbumin was introduced. At position 200 ° the lactalbumin was eluted with 50 mM Tris/HCl, 10mM CaCl$_2$, 2mM cysteine/cystine buffer pH 7.0 at postion 300 ° the column was regenerated with 20 % ethanol dissolved in water. The regenerate was continuously ultra-diafiltrated by a Millipore system using a Biomax 5 filter. As diafiltration buffer a 50 mM Tris/HCl, 10mM CaCl$_2$, pH 7.0 in 6 M GuHCl, 250 mM β-mercaptoethanol and 1.5 M ammonium sulfate was used. The ultra-diafiltrated solution was recycled to the feed and the continuous refolding was performed until steady state conditions were reached.

Example 4

Continuous refolding and separation of native recombinant human alpha-Fetoprotein (rHuAFP), folding intermediates and aggregates

rHuAFP is a complex protein, which contains 16 disulfide bridges. It is produced in E.coli as inclusion body.

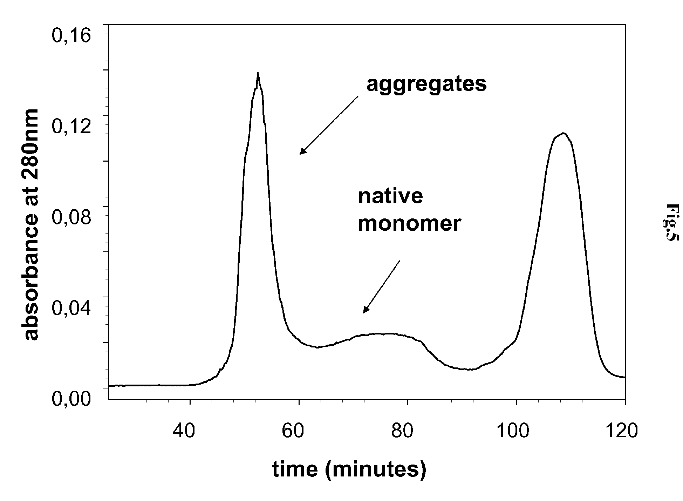

a) Before transferring the process to a combined continuous mode, the refolding and capture step of rHuAFP were tested on conventional chromatography columns packed with Sephacryl 200HR and Q-Sepharose XL from AP biotech (Uppsala, Sweden). The inclusion bodies containing rHuAFP were isolated as described in example 1 and dissolved in 50mM Tris-HCl, pH 8.5, 6M GuHCl and 100mM DTT. The final rHuAFP concentration was about 0.5 mg/ml, the total protein concentration including protein impurities from host cells was approx. 5mg/ml. Refolding of the protein was done by matrix assisted refolding using gel-permeation chromatography. The column was equilibrated with PBS (pH 7.4) at a linear velocity of 11 cm/h. 1ml of the feed solution was loaded onto a column with 2.6 cm i.d. and 26 cm length packed with Sephacryl 200HR. While passing through the column the chaotropic and reducing components were separated from rHuAFP and the protein started to refold (see Fig. 5, which shows matrix-assisted refolding of rHuAFP on Sephacryl 200HR).

After refolding, only 20% of the protein are in the native conformation, the remaining protein consists of stable folding intermediates resulting from non-native disulfide bridges and irreversible aggregates. The fractions were collected and analyzed by SDS-PAGE and Western-blot.

In the next step, the protein was captured by ion-exchange chromatography (Q-Sepharose XL).

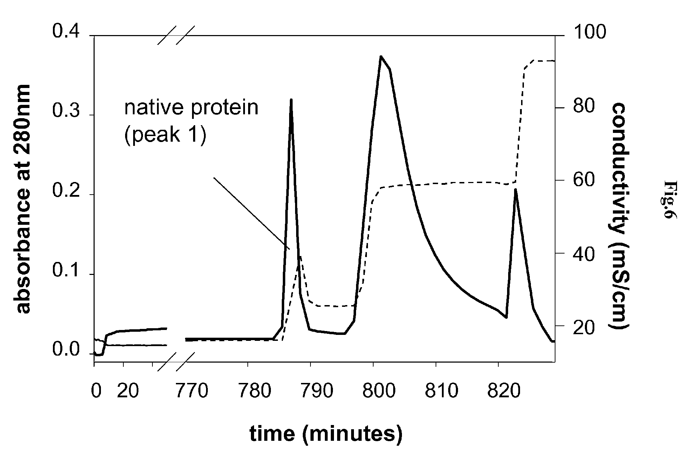

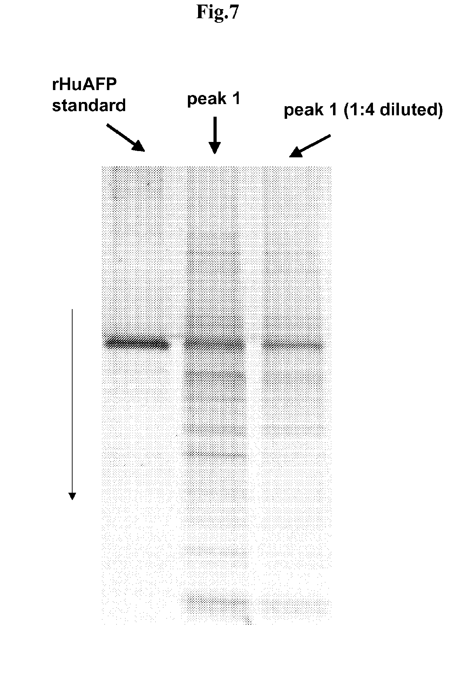

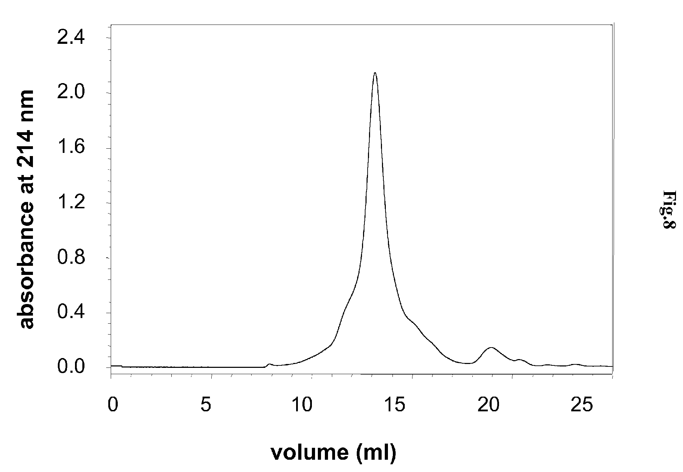

The collected protein fractions were loaded onto an Q-Sepharose XL column (10mm i.d., 50mm height) equilibrated with PBS. Folding intermediates did not interact with the matrix and eluted in the flow through, native rHuAFP was bound onto the resin and eluted in a step gradient with PBS+0.2M NaCl, and aggregates were eluted in a second step gradient with PBS+0.5M NaCl. The chromatogram of the capture step is shown in Fig. 6. The collected native peak fraction (eluate 1) was analyzed by silver-stained SDS-PAGE (Fig. 7) and analytical size exclusion chromatography using a Superdex 200HR column (AP biotech, Uppsala, Sweden) (Fig. 8).

b) For the continuous refolding experiments, the same protein solutions, gels and buffers were used as in a).

Two chromatographic media were packed into a annular chromatography System, PCAC from Prior Separation Technology Götzis, Austria. The lower layer consists of 5cm Q-Sepharose XL and the upper layer of 35cm Sephacryl 200HR.

The system was equilibrated with PBS at a linear velocity of 20 cm/h. The rotation speed of the cylinder was 250°/h.

The feed stream was pumped at the top of the gel bed through a fixed feed nozzle, whose tip was located within the layer of the glass beads. PBS+0.2M NaCl was pumped by another nozzle with a shift of -60 degrees from the feed-nozzle on the top of the gel bed.

In the first gel layer (Sephacryl 200HR) the denaturated and reduced protein started to refold. High molecular weight aggregates were separated from refolded monomeric native rHuAFP and monomeric folding intermediates. After leaving the first gel layer the proteins were captured in the second lower gel layer (Q-Sepharose XL). Native monomeric rHuAFP was eluted continuously with PBS+0.2M NaCl and aggregates were eluted in the salt fraction containing 6M GuHCl and 0.1M DTT.

Samples were drawn and the amount of aggregated protein and native protein were determined by SDS-PAGE.

Example 5

Continuous refolding of α-lactalbumin by ion-exchange chromatography

a) Conditions for refolding of the model protein α-lactalbumin by ion exchange chromatography were optimized in batch mode. A column (0.5 cm i.d.) was packed with DEAE Sepharose 4FF (AP biotech, Uppsala, Sweden). The resulting bed height was 8 cm, which was approximately in the same range as used for the continuous mode. The equilibration buffer was 20mM Tris/HCl, 2mM $CaCl_2$, 2M urea adjusted to pH 8. The elution buffer was the same as the equilibration buffer, supplemented with 0.5M NaCl. As regeneration buffer, either 6M GuHCl containing 100mM monothioglycerol or 0.5M NaOH was used. A total amount of 3mg of denatured and reduced α-lactalbumin was loaded onto DEAE Sepharose 4FF. After washing out the denaturing and reducing agents, the protein was eluted. The collected pool, containing reduced alpha-lactalbumin, was supplemented with cysteine and cystine to final concentrations of 2mM, respectively. After incubation for 6-7 hours in the refolding buffer, about 80% of the initially loaded protein are in native conformation. Under these conditions, the total protein recovery was 90-100%.

Alternatively, 2mM cystine and 2mM cysteine were added to the equilibration and elution buffers. In this case, protein was eluted in its native conformation. The yield of native protein was about 10% and the recovery 80%. In order to simulate conditions required for the continuous process, restrictions concerning flow rate and amount of sample load have been made.

b) Continuous refolding without recycling of aggregates

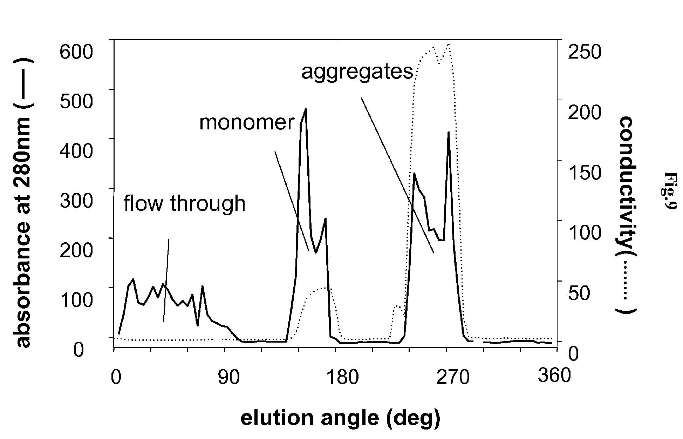

Parameters for the continuous mode were maintained as used in batch experiments. Flow velocities of the different buffers and the angles for application of the buffers were calculated. Solutions of 0.1 mg/ml and 1mg/ml denatured and reduced α-lactalbumin were applied to the pressurized continuous annular chromatograph (PCAC) packed with DEAE Sepharose 4FF. The equilibration buffer (20mM Tris/HCl, 2mM $CaCl_2$, 2M urea) was pumped with a P- 6000 (AP biotech, Uppsala, Sweden) at a flow rate of 22 ml/min through the main inlet port. The load was applied at 0° at a flow rate of 4.2 ml/min with a P-500 pump (AP biotech, Uppsala, Sweden). Elution was effected at 135° with elution buffer (20mM Tris/HCl, 2mM CaCl$_2$, 2M urea, 0.5M NaCl) at a flow rate of 2.1ml/min with a P-500 pump (AP biotech, Uppsala, Sweden). The regeneration solution (6M GuHCl, 100mM monothioglycerol) was pumped with a peristaltic laboratory pump at a flow rate of 1.5ml/min at 222° into the annular chromatograph. After reaching steady state equilibrium, all 90 fractions have been collected two times for 20 minutes. The conductivity was determined and UV adsorption was measured with an external photometer (Hitachi). A representative chromatogram is shown in Figure 9. The fractions in which the protein eluted were determined and samples were taken. An aliquot of 200mM cysteine and 200mM cystine stock solutions were added to each fraction to a final concentration of 2mM. After incubation for 7 hours, the protein content and the folding conformation was determined by reversed phase HPLC. The yield and recovery of native protein was 80% and 95%, respectively.

c) Continuous refolding with recycling of aggregates
Refolding of the model protein can be accelerated by adding the 2mM cysteine and 2mM cystine to the equilibration and elution buffer. The protein regains its native structure during chromatography. Due to faster refolding kinetics, aggregation takes place either to a greater extend on the column. Under these conditions, the protein elutes from the column in its native state. However, most of the protein aggregates during chromatography. The aggregates can be quantitatively removed from the column with 6M GuHCl containing 100mM monothioglycerol as reducing agent. In order to recycle the dissolved aggregates back to the ion exchange resin, the conductivity has to be below 1 mS/cm. Therefore, the dissolved aggregates have to be diafiltrated against 8M urea.

A 0.1mg/ml and 0.5mg/ml solution of denatured and reduced α-lactalbumin was loaded. Regeneration was effected with 6M GuHCl containing 100mM monothiogylcerol as reducing agent. All 90 fractions were collected to determine the fractions containing eluate and regenerate. The fractions containing the regenerate were pooled and diafiltrated against 8M urea until the conductivity was the same as in the feed solution. Ultradifiltration was effected with an tangential flow laboratory ultrafiltration unit (Labscale TFF system, Millipore). Final protein content was determined with reversed phase HPLC. Finally, the diafiltrate was pumped into the feed solution. The yield of native protein increases from 14% without recycling to 78% with recycling of the aggregated protein fraction.

References

1 Horwich, A.L., Neupert, W. and Hartl, F.U. (1990) Protein-catalysed protein folding Trends Biotechnol, 8, 126-131.

2 Noiva, R. (1994) Enzymatic catalysis of disulfide formation Protein Expr Purif, 5, 1-13.

3 Schonbrunner, E.R., Schmid, F.X., Mayer, S., Tropschug, M., Fischer, G. and Takahashi, N. (1992) Peptidyl-prolyl cis-trans isomerase improves the efficiency of protein disulfide isomerase as a catalyst of protein folding catalysis of protein folding by cyclophilins from different species Proc Natl Acad Sci U S A, 89, 4510-4513.

4 Lilie, H., Lang, K., Rudolph, R., Buchner, J., Schonbrunner, E.R., Schmid, F.X., Mayer, S., Tropschug, M., Fischer, G. and Takahashi, N. (1993) Prolyl isomerases catalyze antibody folding in vitro Protein Sci, 2, 1490-1496.

5 Jager, M., Pluckthun, A., Yang, H.P., Zhong, H.N., Zhou, H.M., Lilie, H., Lang, K., Rudolph, R., Buchner, J., Schonbrunner, E.R., Schmid, F.X., Mayer, S., Tropschug, M., Fischer, G. and Takahashi, N. (1997) The rate-limiting steps for the folding of an antibody scFv fragment FEBS Lett, 418, 106-110.

6 Yang, H.P., Zhong, H.N., Zhou, H.M., Lilie, H., Lang, K., Rudolph, R., Buchner, J., Schonbrunner, E.R., Schmid, F.X., Mayer, S., Tropschug, M., Fischer, G. and Takahashi, N. (1997) Catalysis of the refolding of urea denatured creatine kinase by peptidyl-prolyl cis-trans Biochim Biophys Acta, 1338, 147-150.

7 Kane, J.F. and D.L., H. (1988) Formation of recombinant inclusion bodies in Escherichia coli TIBTECH, 6, 95-100.

8 Buchner, J. and Rudolph, R. (1991) Routes to active proteins from transformed microorganisms Current Opinion Biotechnology, 2, 532-538.

9 Halenbeck, R., Kawasaki, E., Wrin, J. and Koths, K. (1989) Renaturation and purification of biologicla active recombinant human macrophage colony-stimulating factor expressed in E.coli Bio/technology, 7, 710-715.

10 Kiefhaber, T., Rudolph, R., Kohler, H.H. and Buchner, J. (1991) Protein aggregation in vitro and in vivo: a quantitative model of the kinetic competition between folding and aggregation Biotechnology (N Y), 9, 825- 829.

11 Lilie, H., Schwarz, E. and Rudolph, R. (1998) Advances in refolding of proteins produced in E. coli Curr Opin Biotechnol, 9, 497-501.

12 Clark, E.D. (2001) Protein refolding for industrial processes Curr Opin Biotechnol, 12, 202-207.

13 Yoshi, H., Furuta, T., Yonehara, T., Ito, D. and Linko, P. (2001) Refolding of denatured and reduced lysozyme with cysteine/cystine red/ox solution in diafiltration, J Chem Eng Japan, 34, 211-215.

14 Buchner, J., Brinkmann, U. and Pastan, I. (1992) Renaturation of a single- chain immunotoxin facilitated by chaperones and protein disulfide isomerase Biotechnology (N Y), 10, 682-685.

15 Carlson, J.D. and Yarmush, M.L. (1992) Antibody assisted protein refolding Biotechnology (N Y), 10, 86-91.

16 Guise, A.D. and Chaudhuri, J.B. (1998) Recovery and reuse of the molecular chaperone GroEL for in vitro protein refolding Biotechnol Prog, 14, 343-346.

17 Kohler, R.J., Preuss, M. and Miller, A.D. (2000) Design of a molecular chaperone-assisted protein folding bioreactor Biotechnol Prog, 16, 671-675.

18 Shimizu, H., Fujimoto, K. and Kawaguchi, H. (2000) Renaturation of reduced ribonuclease A with a microsphere-induced refolding system Biotechnol Prog, 16, 248-253.

19 Katoh, S. and Katoh, Y. (2000) Continuous refolding of lysozyme with fed- batch addition of denatured protein solution 2000, 35, 1119-1124.

20 Phadtare, S., Fisher, M.T. and Yarbrough, L.R. (1994) Refolding and release of tubulins by a functional immobilized groEL column Biochim Biophys Acta, 1208, 189-192.

21 Altamirano, M.M., Golbik, R., Zahn, R., Buckle, A.M. and Fersht, A.R. (1997) Refolding chromatography with immobilized mini-chaperones Proc Natl Acad Sci USA, 94, 3576-3578.

22 Altamirano, M.M., Garcia, C., Possani, L.D. and Fersht, A.R. (1999) Oxidative refolding chromatography: folding of the scorpion toxin Cn5 Nat Biotechnol, 17, 187-191.

23 Preston, N.S., Baker, D.J., Bottomley, S.P. and Gore, M.G. (1999) The production and characterisation of an immobilised chaperonin system Biochim Biophys Acta, 1426, 99-109.

24 Heuer, C., Kniep, H., Falk, T. and Seidel-Morgenstern, A. (1997) Vergleich verschiedener verfahrenstechnischer Konzepte der präparativen Flüssigchromatographie Chemie Ingenieur Technik, 69, 1535-1546.

25 Begovich, J.M., Byers, C.H. and Sisson, W.G. (1983) A high-capacity pressurized continuous chromatograph Sep. Sci. Tech., 18, 1167-1191.

26 Begovich, J.M. and Sisson, W.G. (1984) A rotating annular chromatograph for continuous separations AIChE Journal, 30, 705-710.

27 Bloomingburg, G.F., Carta, G., Bauer, J.S. and Byers, C.H. (1991) Continuous separation of proteins by annular chromatography Ind. Eng. Chem. Res., 30, 1061-1067.

28 Bloomingburg, G.F. and Carta, G. (1994) Separation of protein mixtures by continuous annular chromatography with step elution Chem. Eng. J., 55, B19-B27.

29 Buchacher, A., Iberer, G., Jungbauer, A., Schwinn, H. and Josic, D. (2000) Continuous removal of protein aggregates by annular chromatography Biotechnology Progress, 17, 140-149.

30 Byers, C.H., Sisson, W.G., DeCarli, I.-J.P. and Carta, G. (1989) Pilot-scale studies of sugar separations by continuous chromatography Appl.Biochem.Biotechnol., 20-21, 635-654.

31 Byers, C.H., Sisson, W.G., De Carli II, J.P. and Carta, G. (1990) Sugar separations on a pilot scale by continuous annular chromatography Biotechnol. Prog., 6, 13-20.

32 Canon, R.M., Begovich, J.M. and Sission, W.G. (1980) Pressurized continuous chromatography Separation Science and Technology, 15, 655- 678.

33 Canon, R.M. and Sisson, W.G. (1978) Operation of an improved, continuous annular chromatography. Liqu. Chrom., 1, 427-441.

34 Carta, G., DeCarli, J.P., Byers, C.H. and Sisson, W.G. (1989) Separation of metals by continuous annular chromatography with step elution Chem. Eng. Com., 79, 207-227.

35 Carta, G. and Byers, C.H. (1989) Novel Applications of Continuous Annular Chromatography. New Directions in Sorption technology. Keller, G. E., Yang, R. T., Butterworth.

36 Dalvie, S.K., Gajiwala, K.S. and Baltus, R.E. (1990) Mathematical model of a rotating annular continuous size exclusion chromatograph. In Hamel, J.-F.P., Hunter, J.B. and Sikdar, S.K. (eds.), Downstream Processing and Bioseparation, pp. 268-284.

37 Reissner, K., Bart, H.J., Prior, A., Wolfgang, J. and Byers, C.H. (1997) Preparative desalting of bovine serum albumin by continuous annular chromatography J. Chromatogr., 763, 49-56.

38 Scott, C.D., Spence, R.D. and Sisson, W.G. (1976) Pressurized, annular chromatograph for continuous separations J. Chromatogr., 126, 381-400.

39 Uretschläger, A., Einhauer, A. and Alois, J. (2000) Continuous separation of green fluorescent protein by annular chromatography J. Chrom. A, in press.

40 Wolfgang, J., Prior, A., Bart, H.J., Messenböck, R.C. and Byers, C.H. (1997) Continuous separation of carbohydrates by ion-exchange chromatography Sep. Sci. Tech., 32, 71-82.

41 Yamanishi, Y. and Yonemoto, T. (1997) Complete separation of amino acids using continuous rotating annular ion-exchange chromatography with partial recycle of effluent In. Eng. Chem. Res., 36, 3809-3814.

42 Tagahashi, Y. and Goto, S. (1994) Continuous separation of fructooligosaccharides using an annular chromatograph Sep. Sci. Techn., 29**.**

43 Takahashi, Y. and Goto, S. (1991) Continuous separation using an annular chromatograph with non isocratic elution J. Chem. Eng., 24, 121-123.

44 Takahashi, Y. and Goto, S. (1991) Continuous separations of amino acids by using an annular chromatograph with rotating inlet and outlet Sep. Sci. Tech., 26, 1-13.

45 Takahashi, Y. and Goto, S. (1991) Continuous concentration of single component using an annular chromatograph J. Chem. Eng., 24, 460-465.

46 Takahashi, Y. and Goto, S. (1992) Continuous separation and concentration of proteins using an annular chromatograph J. Chem. Eng., 25, 403-407.

47 Takahashi, Y. and Goto, S. (1994) Continuous separation of fructooligosaccharides using an annular chromatograph Sep. Sci. Techn., 29, 1311-1318.

48 Sisson, W.G., Begovich, J.M., Byers, C.H. and Scott, C.D. (1988) Continuous chromatography CHEMTECH, 498-501.

49 Sisson, W.G., Scott, C.D., Begovich, J.M. and Byers, C.H. (1989) Application of continuous annular chromatography to size exclusion separations Prep. Chrom., 1 (2), 139-162.

50 Goto, M. and Goto, S. (1987) Continuous separation using an annular chromatograph with rotating inlet and outlet J. Chem. Eng., 20, 598-603.

51 Kitakawa, A., Yamanishi, Y., Yonemoto, T. and Tadaki, T. (1995) Modeling and simulation of continuous rotating annular ion-exchange chromatography for separation of amino acids Sep. Sci. Tech., 30, 3089-3110.

52 Fox, J.B., Calhoun, R.C. and Eglinton, W.J. (1969) Continuous chromatography apparatus, I. Construction J. Chromatogr. A, 43, 48-54.

53 Fox, J.B. and Nicholas, R.A. (1969) Continuous chromatography apparatus, III. Application J. Chromatogr. A, 43, 61-65.

54 Fox, J.B. (1969) Continuous chromatography apparatus, II. Operation J. Chromatogr. A, 43, 55-60.

55 Broughton, D.B. (1984) Production scale separations of liquid muixtures by simulated moving-bed technology Sep. Sci. Technol., 19, 723-736.

56 Schulte, M. and Strube, J. (2001) Preparative enantioseparation by simulated moving bed chromatography J Chromatogr A, 906, 399-416.

57 Hidajat, K., Ching, C. and Ruthven, D.M. (1986) Simulated countercurrent adsoption processes: A threroetical analysis of the effect of subdividing the adsorbent bed Chem. Eng. Sci., 41, 2953-2956.

58 Clark, E.D., Schwarz, E. and Rudolph, R. (1999) Inhibition of aggregation side reactions during in vitro protein folding Methods Enzymol., 309, 217- 236.

59 Martin, A.V.P. (1949) Summarizing paper of a general discussion on chromatographic analysis Faraday Soc., 7, 332-336.

60 Bosmenu, R., Semeniuk, D. and Murgita, R.A. (1997) Purification and characterization of human and mouse recombinant alpha-fetoprotein expressed in Escherichia coli Protein Expr. Purif., 10, 10-26

61 Feng, W., Graumann, K., Hahn, R. and Jungbauer, A. (1999) Affinity chromatography of human estrogen receptor-$\alpha$ expressed in Sacharomyces cerevisiae Combination of heparin- and 17$\beta$-estradiol-affinity chromatography J Chromatogr A, 852, 161-173

## Claims

1. A method for obtaining a biologically active recombinant protein by reconstituting the protein from a denatured state to its active form, wherein a feed solution containing the recombinant protein of interest in its denatured form and/or in biologically inactive intermediate forms is subject to a chromatographic separation process, in which said protein is reconstituted under conditions that promote refolding of the protein and the intermediate forms are separated from the refolded protein, **characterized in that** the denatured form and/or the inactive intermediate forms of the protein are separated from the refolded protein in a continuous or quasi-continuous chromatographic method.

2. The method of claim 1, wherein the intermediate forms that have been separated from the refolded protein are reintroduced into the feed solution and thus undergo at least one more reconstitution process.

3. The method of claim 1 or 2, wherein the separation process is selected from ion-exchange chromatography, affinity chromatography, hydrophobic interaction chromatography, reversed phase chromatography, covalent chromatography, size exclusion chromatography and adsorption chromatography.

4. The method of claim 3, wherein the separation process is operated continuously in the form of annular chromatography.

5. The method of claim 3, wherein the separation process is operated quasi-continuously in the form of a simulated moving bed chromatography.

6. The method of claim 3, wherein the separation process is operated quasi-continuously in the form of a carrousel chromatography.

EP 2 298 788 A1

Fig.1

14

Fig.2

Fig.3

Fig.4A

Fig.4B

Fig.4C

Fig.5

Fig.6

Fig.7

Fig.8

Fig.9

Fig.10

European Patent Office

**EUROPEAN SEARCH REPORT**

Application Number

EP 10 18 0934

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | ALTAMIRANO, M. ET AL.: "Refolding chromatography with immobilized mini-chaperones", PROC. NATL. ACAD. SCI. USA, vol. 94, April 1997 (1997-04), page 3576-3578, XP002147144, | 1,3 | INV. C07K1/113 C07K1/16 C07K1/18 C07K1/20 C07K1/22 C07K1/36 |
| Y | * Abstract, Figure, refolding capacity of Mini-Chaperone Agarose, (Materials and Methods), Refolding Chromatography (Results and Discussion). * | 2,4-6 | |
| X,D | KOHLER, R. ET AL.: "Design of a Molecular Chaperone-Assisted Protein folding Bioreactor", BIOTECH. PROG., vol. 16, 1 January 2000 (2000-01-01), page 671-675, XP002188674, | 1,3 | |
| Y | * Abstract, Results and Discussion. Figs.1-3 * | 2,4-6 | |
| Y,D | KATOH S., ET KATOH Y.: "Continuous refolding of lysozyme with fed-batch addition of denatured protein solution", PROCESS BIOCHEMISTRY, vol. 35, June 2000 (2000-06), pages 1119-1124, XP002224937, * Introduction, Materials and methods, 2.4.3, Fig. 2, results and discussion. * | 1-6 | TECHNICAL FIELDS SEARCHED (IPC) C07K |
| Y | US 4 511 502 A (OGEZ JOHN R ET AL) 16 April 1985 (1985-04-16) * See entire document, esp. col. 3, lines 12-67, Scheme 2 and Examples. * | 1-6 | |

-/--

The present search report has been drawn up for all claims

| Place of search | Date of completion of the search | Examiner |
|---|---|---|
| Munich | 25 January 2011 | Mueller, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

# EUROPEAN SEARCH REPORT

Application Number

EP 10 18 0934

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 5 310 688 A (COLTON CLARK K ET AL) 10 May 1994 (1994-05-10) * See Abstract, Fig.1, Background, "The elution Step in Affinity Separations", Examples. * ----- | 1-6 | |
| A | EP 0 360 937 A (CETUS CORP) 4 April 1990 (1990-04-04) * the whole document * ----- | 1-6 | |

TECHNICAL FIELDS
SEARCHED    (IPC)

The present search report has been drawn up for all claims

| Place of search | Date of completion of the search | Examiner |
|---|---|---|
| Munich | 25 January 2011 | Mueller, Frank |

EPO FORM 1503 03.82 (P04C01)

**ANNEX TO THE EUROPEAN SEARCH REPORT
ON EUROPEAN PATENT APPLICATION NO.**                EP 10 18 0934

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2011

| Patent document cited in search report | | Publication date | Patent family member(s) | | Publication date |
|---|---|---|---|---|---|
| US 4511502 | A | 16-04-1985 | NONE | | |
| US 5310688 | A | 10-05-1994 | NONE | | |
| EP 0360937 | A | 04-04-1990 | AT | 101654 T | 15-03-1994 |
| | | | AU | 631356 B2 | 26-11-1992 |
| | | | AU | 2284188 A | 29-03-1990 |
| | | | CA | 1340586 C | 08-06-1999 |
| | | | DE | 3887889 D1 | 24-03-1994 |
| | | | DE | 3887889 T2 | 25-08-1994 |
| | | | IE | 62661 B1 | 22-02-1995 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9845699 A
- WO 9928740 A
- WO 01388866 A
- EP 1134581 A

### Non-patent literature cited in the description

- **Horwich, A.L. ; Neupert, W. ; Hartl, F.U.** Protein-catalysed protein folding. *Trends Biotechnol,* 1990, vol. 8, 126-131
- **Noiva, R.** Enzymatic catalysis of disulfide formation. *Protein Expr Purif,* vol. 5, 1-13
- **Schonbrunner, E.R. ; Schmid, F.X. ; Mayer, S. ; Tropschug, M. ; Fischer, G. ; Takahashi, N.** Peptidyl-prolyl cis-trans isomerase improves the efficiency of protein disulfide isomerase as a catalyst of protein folding catalysis of protein folding by cyclophilins from different species. *Proc Natl Acad Sci U S A,* 1992, vol. 89, 4510-4513
- **Lilie, H. ; Lang, K. ; Rudolph, R. ; Buchner, J. ; Schonbrunner, E.R. ; Schmid, F.X. ; Mayer, S. ; Tropschug, M. ; Fischer, G. ; Takahashi, N.** Prolyl isomerases catalyze antibody folding in vitro. *Protein Sci,* 1993, vol. 2, 1490-1496
- **Jager, M. ; Pluckthun, A. ; Yang, H.P. ; Zhong, H.N. ; Zhou, H.M. ; Lilie, H. ; Lang, K. ; Rudolph, R. ; Buchner, J. ; Schonbrunner, E.R.** The rate-limiting steps for the folding of an antibody scFv fragment. *FEBS Lett,* vol. 418, 106-110
- **Yang, H.P. ; Zhong, H.N. ; Zhou, H.M. ; Lilie, H. ; Lang, K. ; Rudolph, R. ; Buchner, J. ; Schonbrunner, E.R. ; Schmid, F.X. ; Mayer, S.** Catalysis of the refolding of urea denatured creatine kinase by peptidyl-prolyl cis-trans. *Biochim Biophys Acta,* 1997, vol. 1338, 147-150
- **Kane, J.F. ; D.L., H.** Formation of recombinant inclusion bodies in Escherichia coli. *TIBTECH,* 1998, vol. 6, 95-100
- **Buchner, J. ; Rudolph, R.** Routes to active proteins from transformed microorganisms. *Current Opinion Biotechnology,* 1991, vol. 2, 532-538
- **Halenbeck, R. ; Kawasaki, E. ; Wrin, J. ; Koths, K.** Renaturation and purification of biologicla active recombinant human macrophage colony- stimulating factor expressed in E.coli. *Bio/technology,* 1989, vol. 7, 710-715

- **Kiefhaber, T. ; Rudolph, R. ; Kohler, H.H. ; Buchner, J.** Protein aggregation in vitro and in vivo: a quantitative model of the kinetic competition between folding and aggregation. *Biotechnology (N Y),* 1991, vol. 9, 825-829
- **Lilie, H. ; Schwarz, E. ; Rudolph, R.** Advances in refolding of proteins produced in E. coli. *Curr Opin Biotechnol,* 1998, vol. 9, 497-501
- **Clark, E.D.** Protein refolding for industrial processes. *Curr Opin Biotechnol,* 2001, vol. 12, 202-207
- **Yoshi, H. ; Furuta, T. ; Yonehara, T. ; Ito, D. ; Linko, P.** Refolding of denatured and reduced lysozyme with cysteine/cystine red/ox solution in diafiltration. *J Chem Eng Japan,* 2001, vol. 34, 211-215
- **Buchner, J. ; Brinkmann, U. ; Pastan, I.** Renaturation of a single- chain immunotoxin facilitated by chaperones and protein disulfide isomerase. *Biotechnology (N Y),* 1992, vol. 10, 682-685
- **Carlson, J.D. ; Yarmush, M.L.** Antibody assisted protein refolding. *Biotechnology (N Y),* 1992, vol. 10, 86-91
- **Guise, A.D. ; Chaudhuri, J.B.** Recovery and reuse of the molecular chaperone GroEL for in vitro protein refolding. *Biotechnol Prog,* 1998, vol. 14, 343-346
- **Kohler, R.J. ; Preuss, M. ; Miller, A.D.** Design of a molecular chaperone-assisted protein folding bioreactor. *Biotechnol Prog,* 2000, vol. 16, 671-675
- **Shimizu, H. ; Fujimoto, K. ; Kawaguchi, H.** Renaturation of reduced ribonuclease A with a microsphere-induced refolding system. *Biotechnol Prog,* 2000, vol. 16, 248-253
- **Katoh, S. ; Katoh, Y.** *Continuous refolding of lysozyme with fed- batch addition of denatured protein solution,* 2000, vol. 35, 1119-1124
- **Phadtare, S. ; Fisher, M.T. ; Yarbrough, L.R.** Refolding and release of tubulins by a functional immobilized groEL column. *Biochim Biophys Acta,* 1994, vol. 1208, 189-192

- **Altamirano, M.M. ; Golbik, R. ; Zahn, R. ; Buckle, A.M. ; Fersht, A.R.** Refolding chromatography with immobilized mini-chaperones. *Proc Natl Acad Sci USA,* 1997, vol. 94, 3576-3578
- **Altamirano, M.M. ; Garcia, C. ; Possani, L.D. ; Fersht, A.R.** Oxidative refolding chromatography: folding of the scorpion toxin Cn5. *Nat Biotechnol,* 1999, vol. 17, 187-191
- **Preston, N.S. ; Baker, D.J. ; Bottomley, S.P. ; Gore, M.G.** The production and characterisation of an immobilised chaperonin system. *Biochim Biophys Acta,* 1999, vol. 1426, 99-109
- **Heuer, C. ; Kniep, H. ; Falk, T. ; Seidel-Morgenstern, A.** Vergleich verschiedener verfahrenstechnischer Konzepte der präparativen Flüssigchromatographie. *Chemie Ingenieur Technik,* 1997, vol. 69, 1535-1546
- **Begovich, J.M. ; Byers, C.H. ; Sisson, W.G.** A high-capacity pressurized continuous chromatograph. *Sep. Sci. Tech.,* 1983, vol. 18, 1167-1191
- **Begovich, J.M. ; Sisson, W.G.** A rotating annular chromatograph for continuous separations. *AIChE Journal,* 1984, vol. 30, 705-710
- **Bloomingburg, G.F. ; Carta, G. ; Bauer, J.S. ; Byers, C.H.** Continuous separation of proteins by annular chromatography. *Ind. Eng. Chem. Res.,* 1991, vol. 30, 1061-1067
- **Bloomingburg, G.F. ; Carta, G.** Separation of protein mixtures by continuous annular chromatography with step elution. *Chem. Eng. J.,* 1994, vol. 55, B19-B27
- **Buchacher, A. ; Iberer, G. ; Jungbauer, A. ; Schwinn, H. ; Josic, D.** Continuous removal of protein aggregates by annular chromatography. *Biotechnology Progress,* 2000, vol. 17, 140-149
- **Byers, C.H. ; Sisson, W.G. ; DeCarli, I.-J.P. ; Carta, G.** Pilot-scale studies of sugar separations by continuous chromatography. *Appl.Biochem.Biotechnol.,* 1989, vol. 20-21, 635-654
- **Byers, C.H. ; Sisson, W.G. ; De Carli II, J.P. ; Carta, G.** Sugar separations on a pilot scale by continuous annular chromatography. *Biotechnol. Prog.,* 1990, vol. 6, 13-20
- **Canon, R.M. ; Begovich, J.M. ; Sission, W.G.** Pressurized continuous chromatography. *Separation Science and Technology,* 1980, vol. 15, 655-678
- **Canon, R.M. ; Sisson, W.G.** Operation of an improved, continuous annular chromatography. *Liqu. Chrom.,* 1978, vol. 1, 427-441
- **Carta, G. ; DeCarli, J.P. ; Byers, C.H. ; Sisson, W.G.** Separation of metals by continuous annular chromatography with step elution. *Chem. Eng. Com.,* 1989, vol. 79, 207-227
- **Carta, G. ; Byers, C.H. ; Keller, G. E. ; Yang, R. T.** Novel Applications of Continuous Annular Chromatography. *New Directions in Sorption technology,* 1989
- Mathematical model of a rotating annular continuous size exclusion chromatograph. **Dalvie, S.K. ; Gajiwala, K.S. ; Baltus, R.E.** Downstream Processing and Bioseparation. 1990, 268-284
- **Reissner, K. ; Bart, H.J. ; Prior, A. ; Wolfgang, J. ; Byers, C.H.** Preparative desalting of bovine serum albumin by continuous annular chromatography. *J. Chromatogr.,* 1997, vol. 763, 49-56
- **Scott, C.D. ; Spence, R.D. ; Sisson, W.G.** Pressurized, annular chromatograph for continuous separations. *J. Chromatogr.,* 1976, vol. 126, 381-400
- **Uretschläger, A. ; Einhauer, A. ; Alois, J.** Continuous separation of green fluorescent protein by annular chromatography. *J. Chrom. A,* 2000
- **Wolfgang, J. ; Prior, A. ; Bart, H.J. ; Messenböck, R.C. ; Byers, C.H.** Continuous separation of carbohydrates by ion-exchange chromatography. *Sep. Sci. Tech.,* 1997, vol. 32, 71-82
- **Yamanishi, Y. ; Yonemoto, T.** Complete separation of amino acids using continuous rotating annular ion-exchange chromatography with partial recycle of effluent. *In. Eng. Chem. Res.,* 1997, vol. 36, 3809-3814
- **Tagahashi, Y. ; Goto, S.** Continuous separation of fructooligosaccharides using an annular chromatograph. *Sep. Sci. Techn.,* 1994, vol. 29
- **Takahashi, Y. ; Goto, S.** Continuous separation using an annular chromatograph with non isocratic elution. *J. Chem. Eng.,* 1991, vol. 24, 121-123
- **Takahashi, Y. ; Goto, S.** Continuous separations of amino acids by using an annular chromatograph with rotating inlet and outlet. *Sep. Sci. Tech.,* 1991, vol. 26, 1-13
- **Takahashi, Y. ; Goto, S.** Continuous concentration of single component using an annular chromatograph. *J. Chem. Eng.,* 1991, vol. 24, 460-465
- **Takahashi, Y. ; Goto, S.** Continuous separation and concentration of proteins using an annular chromatograph. *J. Chem. Eng.,* 1992, vol. 25, 403-407
- **Takahashi, Y. ; Goto, S.** Continuous separation of fructooligosaccharides using an annular chromatograph. *Sep. Sci. Techn.,* 1994, vol. 29, 1311-1318
- **Sisson, W.G. ; Begovich, J.M. ; Byers, C.H. ; Scott, C.D.** Continuous chromatography. *CHEMTECH,* 1988, 498-501
- **Sisson, W.G. ; Scott, C.D. ; Begovich, J.M. ; Byers, C.H.** Application of continuous annular chromatography to size exclusion separations. *Prep. Chrom.,* 1989, vol. 1 (2), 139-162
- **Goto, M. ; Goto, S.** Continuous separation using an annular chromatograph with rotating inlet and outlet. *J. Chem. Eng.,* 1987, vol. 20, 598-603

- **Kitakawa, A. ; Yamanishi, Y. ; Yonemoto, T. ; Tadaki, T.** Modeling and simulation of continuous rotating annular ion-exchange chromatography for separation of amino acids. *Sep. Sci. Tech.,* 1995, vol. 30, 3089-3110
- **Fox, J.B. ; Calhoun, R.C. ; Eglinton, W.J.** Continuous chromatography apparatus, I. Construction. *J. Chromatogr. A,* vol. 43, 48-54
- **Fox, J.B. ; Nicholas, R.A.** Continuous chromatography apparatus, III. *Application J. Chromatogr. A,* 1969, vol. 43, 61-65
- **Fox, J.B.** Continuous chromatography apparatus, II. *Operation J. Chromatogr. A,* 1969, vol. 43, 55-60
- **Broughton, D.B.** Production scale separations of liquid muixtures by simulated moving-bed technology. *Sep. Sci. Technol.,* 1984, vol. 19, 723-736
- **Schulte, M. ; Strube, J.** Preparative enantioseparation by simulated moving bed chromatography. *J Chromatogr A,* 2001, vol. 906, 399-416
- **Hidajat, K. ; Ching, C. ; Ruthven, D.M.** Simulated countercurrent adsoption processes: A threoretical analysis of the effect of subdividing the adsorbent bed. *Chem. Eng. Sci.,* 1986, vol. 41, 2953-2956
- **Clark, E.D. ; Schwarz, E. ; Rudolph, R.** Inhibition of aggregation side reactions during in vitro protein folding. *Methods Enzymol.,* 1999, vol. 309, 217-236
- **Martin, A.V.P.** Summarizing paper of a general discussion on chromatographic analysis. *Faraday Soc.,* 1949, vol. 7, 332-336
- **Bosmenu, R. ; Semeniuk, D. ; Murgita, R.A.** Purification and characterization of human and mouse recombinant alpha-fetoprotein expressed in Escherichia coli. *Protein Expr. Purif.,* 1997, vol. 10, 10-26
- **Feng, W. ; Graumann, K. ; Hahn, R. ; Jungbauer, A.** Affinity chromatography of human estrogen receptor-$\alpha$ expressed in Sacharomyces cerevisiae Combination of heparin- and 17$\beta$-estradiol-affinity chromatography. *J Chromatogr A,* 1999, vol. 852, 161-173